# EUROPÄISCHE PATENTANMELDUNG

(11) **EP 2 716 622 A1**
(43) Veröffentlichungstag der Anmeldung: **09.04.2014**
(21) Anmeldenummer: 12006926.5
(22) Anmeldetag: 05.10.2012
(51) Int. Cl.: C07C 5/48, C07C 11/04

(54) **Reaktoreinrichtung und Verfahren zur oxidativen Dehydrierung von Alkanen**

(71) Anmelder: Linde Aktiengesellschaft, 80331 Munich (DE)
(72) Erfinder: Hofmann, Karl-Heinz, Dr., 82110 Germering (DE); Meiswinkel, Andreas, Dr., 81479 München (DE); Thaller, Christian, 80687 München (DE); Zander, Hans-Jörg, Dr., 81479 München (DE)
(74) Vertreter: Zahn, Christoph

(57) **Zusammenfassung**

Die Erfindung betrifft ein Verfahren zur oxidativen Dehydrierung von Alkanen, insbesondere von Ethan zu Ethylen, bei dem ein Einsatzstoffstrom (E) enthaltend ein Alkan, insbesondere Ethan, in einer Reaktoreinrichtung (1) einem Katalysator (K) zugeführt wird, wobei durch oxidatives Dehydrieren des Alkans mit Sauerstoff in Gegenwart des Katalysators (K) ein alkenhaltiger Produktstoffstrom (P) erzeugt wird, und wobei ein Verdünnungsmittel in die Reaktoreinrichtung (1) eingeleitet wird, das inert ist oder zumindest eine inerte Komponente aufweist.

## Beschreibung

Die Erfindung betrifft ein Verfahren zur oxidativen Dehydrierung von Alkanen zu dem jeweils korrespondierenden Alken.

Ein bekanntes Verfahren zu Herstellung von Alkenen (Olefinen), wie z.B. Ethylen und Propylen, ist das Steamcracken von unterschiedlichen Kohlenwasserstoffen. In Frage kommen hier insbesondere Ethan, Propan, Naphtha, Gasöle und andere schwere Einsätze. Ein Nachteil des Steamcracking-Verfahrens besteht darin, dass insbesondere bei schwereren Einsätzen weitere Nebenprodukte anfallen, die vom gewünschten (Olefin)produkt abzutrennen sind. Ferner ist das Steamcracken ein energieintensives Verfahren, das bei vergleichsweise hohen Temperaturen im Bereich von ca. 800°C bis 900°C erfolgt. Bei diesem Verfahren werden stets auch Acetylenverbindungen gebildet (z.B. Acetylen, Methylacetylen, Propadien), die eine aufwendige separate Abtrennung durch z.B. Extraktion oder Selektivhydrierung erfordern.

Ein grundsätzlich bekanntes Verfahren als Alternative zur Herstellung von C2-C4-Olefinen, insbesondere Ethylen, ist die oxidative Dehydrierung der korrespondierenden Alkane in Gegenwart eines Katalysators. Prinzipbedingt entstehen hierbei weniger Nebenprodukte.

Ein derartiges Verfahren zur oxidativen Dehydrierung wird z.B. in der WO2010/115108A1 beschrieben, wobei nach der oxidativen Dehydrierung im Produktstoffstrom enthaltener, unreagierter Sauerstoff an einem entsprechenden Katalysator verbrannt wird. Weiterhin wird in der US 6,518,476 B1 als Katalysator für die oxidative Dehydrierung insbesondere ein nicht-stöchiometrischer seltene Erden Oxycarbonat-Katalysator vorgeschlagen. Ferner offenbart die WO2010/115099A1 die Herstellung eines Katalysators für die oxidative Dehydrierung, der neben Mo, V zumindest Nb und/oder Ta, sowie zumindest eines der Elemente Te, Ga, Pd, W, Bi und Al sowie Ni und/oder Sb aufweisen kann. Gemäß EP167109B1 wird als Katalysator bei der Herstellung von Ethylen aus Ethan durch oxidative Dehydrierung u.a. ein MoVNbSbTeMn-Oxid-Katalysator vorgeschlagen. Weiterhin betrifft die US20120016171A1 einen VMoNbTeMe-Oxid-Katalysator, wobei Me aus der Gruppe umfassend Ta, Ti, W, Hf, Zr, Sb stammt. Schließlich schlägt die US20090292153A1 vor, die oxidative Dehydrierung in Gegenwart eines Katalysators sowie von reduzierbaren Metalloxiden vorzunehmen.

Bei der oxidativen Dehydrierung von Alkanen ist weiterhin beachtlich, dass verfahrensmäßig Gemische aus Kohlenwasserstoffen und Sauerstoff zu handhaben sind, die das Beherrschen und/oder Vermeiden von Explosionsbereichen notwendig machen. Weiterhin ist eine leichte Abtrennung bei wirtschaftlicher Verfahrensführung zu gewährleisten. Insbesondere auch Umweltaspekte, wie die effiziente Nutzung von Ressourcen, die Minimierung des Energieverbrauches oder die Reduktion von Emissionen spielen hierbei eine Rolle.

Hiervon ausgehend ist daher ein Verfahren zur oxidativen Dehydrierung wünschenswert, bei dem die Vermeidung besagter Explosionsbereiche in effektiver Weise möglich ist.

Dieses Problem wird durch ein Verfahren mit den Merkmalen des Anspruchs 1 gelöst.

Danach wird bei dem erfindungsgemäßen Verfahren ein Einsatzstoffstrom, der ein Alkan sowie Sauerstoff enthält, in einer Reaktoreinrichtung einem Katalysator zugeführt und Alkan oxidativ unter Entstehung eines alkenhaltigen Produktstoffstromes in Gegenwart des Katalysators dehydriert (z.B. Ethan zu Ethylen, Propan zu Propylen oder Butan zu Butylen), wobei ein Verdünnungsmittel in die Reaktoreinrichtung eingeleitet wird, das inert ist oder zumindest eine inerte Komponente aufweist.

Das Verdünnungsmittel wird bevorzugt eingesetzt, um die Wärmetönung der Reaktion zu beherrschen. Insbesondere soll die oxidative Dehydrierung außerhalb eines Reaktionsbereiches durchgeführt werden.

Bevorzugt wird Wasserdampf, Stickstoff und/oder Luft als Verdünnungsmittel verwendet. Die Luft kann dabei aus der Umgebung genommen werden und normale atmosphärische Sauerstoff- und Stickstoffkonzentrationen aufweisen. Des Weiteren kann der Sauerstoff in besagter Luft an- oder abgereichert sein. Weiterhin kann der Stickstoff in besagter Luft an- oder abgereichert sein.

Die Verwendung von Dampf als Verdünnungsmittel ist insbesondere von Vorteil, da der Dampf zum einen leicht und günstig erzeugt werden kann, und zum anderen auf einfache Weise durch Kondensation abgeschieden werden kann. Des Weiteren kann dabei zugleich eine Reinigung bzw. ein Auswaschen von Verunreinigungen, wie z.B. Säuren und anderen wasserlöslichen Verbindungen, z.B. Carbonylverbindungen, erfolgen. Die Dampfverdünnung bedingt darüberhinaus lediglich geringe Investitionskosten und kann sogar aktivitäts- und/oder selektivitätsfördernd sein.

Bei Verwendung eines Inertmediums wie z.B. Stickstoff zur Verdünnung in der Reaktoreinrichtung können mit Vorteil Explosionsgrenzen eingehalten werden, d.h., explosionsfähige Atmosphären vermieden werden, und zwar sowohl in der Reaktoreinrichtung als auch im Trennteil stromab der Reaktoreinrichtung. Dabei kann der Betrieb der Anlage außerhalb von Explosionsbereichen auch bei erhöhtem Druck erfolgen. Weiterhin kann Stickstoff durch Einspeisung von Luft gewonnen werden und es ist keine eigenständige Luftzerlegung notwendig, um Sauerstoff und/oder Stickstoff zu gewinnen. Dies ermöglicht weiterhin die Nutzung von Luft als Oxidationsmittel, so dass kein Umgang mit reinem Sauerstoff erforderlich ist. Besondere Vorteile ergeben sich ferner aus der Kombination von Stickstoff bzw. Inertmedium und Dampf als Verdünnungsmittel.

Bevorzugt wird beim Anfahren der Anlage, d.h., beim Einleiten der oxidativen Dehydrierung, Wasserdampf oder Stickstoff oder ein Gemisch aus Wasserdampf oder Stickstoff als Verdünnungsmittel in die Reaktoreinrichtung gegeben und sodann besagtes Alkan und als Oxidationsmittel der Sauerstoff oder den Sauerstoff enthaltene Luft hinzugegeben, wobei Sauerstoff oder Stickstoff in besagter Luft jeweils an- oder abgereichert sein kann.

Das beim Anfahren hinzugegebene Verdünnungsmittel kann nach dem Anfahren weiterhin in die Reaktoreinrichtung gegeben werden oder zumindest teilweise oder vollständig durch ein anderes Verdünnungsmittel (z.B. Wasserdampf, Stickstoff und/oder Luft) ersetzt werden.

So wird z.B. in einer Variante des erfindungsgemäßen Verfahrens anstelle von reinem Sauerstoff Luft zur Bereitstellung von Sauerstoff als Oxidationsmittel in die Reaktoreinrichtung eingespeist (siehe oben). Durch Reaktion des Sauerstoffes verbleiben Stickstoff und inerte Komponenten aus der Luft im Produktstoffstrom. Der Stickstoff und Inerte werden bevorzugt abgetrennt und als Verdünnungsmittel in die Reaktoreinrichtung zurückgeführt. Bei der Verwendung von Luft als Oxidationsmittel bzw. Verdünnungsmittel wird Stickstoff bei Inbetriebnahme der Anlage, d.h., beim Anfahren, in die Reaktoreinrichtung eingespeist, wobei Stickstoff bevorzugt lediglich beim Anfahren in die Reaktoreinrichtung eingespeist wird und sodann durch Luft und/oder Wasserdampf ersetzt wird.

Je nach Erfordernis kann eine zusätzliche Einspeisung von reinem N₂ in die Reaktoreinrichtung erfolgen, um die Sauerstoffkonzentration gering zu halten und/oder um hohe Purgeraten zu ermöglichen.

Besonders bevorzugt wird grundsätzlich der Anteil des Verdünnungsmittels im Einsatzstoffstrom so eingestellt, dass sich in keinem Bereich der Reaktoreinrichtung und/oder nachfolgender Apparate (z.B. Zerlegungsteil) eine explosionsfähige Atmosphäre bildet.

Bevorzugt wird die oxidative Dehydrierung in der Reaktoreinrichtung bei einem Druck von 0,5 bar bis 35 bar, bevorzugt 1 bar bis 15 bar, besonders bevorzugt 1 bar bis 5 bar durchgeführt.

Bevorzugt wird die oxidative Dehydrierung weiterhin in der Reaktoreinrichtung bei einer Temperatur von 250°C bis 650°C, bevorzugt 280°C bis 550°C, besonders bevorzugt 350°C bis 480°C durchgeführt.

Bevorzugt handelt es sich bei dem Alkan um ein C2 bis C6-Alkan, besonders bevorzugt um Ethan.

Besonders bevorzugt wird als Katalysator ein MoVTeNbOₓ-Katalysator verwendet, wobei jedoch grundsätzlich ein beliebiger Katalysator mit ausreichenden Aktivitäts- und Selektivitätseigenschaften verwendet werden kann, der unter den gegebenen Reaktionsbedingungen stabil ist.

Weiterhin ist in einer Variante des erfindungsgemäßen Verfahrens vorgesehen, dass der Katalysator mit einem Inertmaterial verdünnt ist.

Bevorzugt weist die Reaktoreinrichtung zumindest ein den Katalysator aufweisendes Festbett auf, wobei vorzugsweise das mindestens eine Festbett aus zumindest einer Mehrzahl an jenen Katalysator aufweisenden ersten Partikeln gebildet ist. In einer Variante des erfindungsgemäßen Verfahrens weisen dabei jene ersten Partikel auch das Inertmaterial auf. Alternativ hierzu ist vorgesehen, dass das mindestens eine Festbett zum Verdünnen des Katalysators eine Mehrzahl an mit den ersten Partikeln vermischte zweite Partikel aufweist, die aus dem Inertmaterial gebildet sind.

Bei den besagten ersten und zweiten Partikeln handelt es sich um makroskopische Partikel, d.h. nicht um einzelne atomare oder molekulare Teilchen. Vorzugsweise weisen diese Partikel eine Korngröße auf, die größer oder gleich einem Mikrometer ist, bevorzugt größer oder gleich 100 Mikrometern, besonders bevorzugt größer oder gleich 1 mm. Die Partikel bilden insbesondere Schüttfüllkörper.

Bevorzugt wird der Grad der Verdünnung des Katalysators mittels des Inertmaterials so bemessen, dass die Reaktionstemperatur bei isothermer Reaktionsführung im Reaktorrohr so hoch gehalten werden kann, dass größtmögliche Umsätze und Selektivitäten erreicht werden, ohne dass es zu einem Durchgehen der Reaktion führt. Bei adiabatischer Prozessführung (insbesondere bei mehreren Festbetten) wird der Grad der Verdünnung des aktiven Katalysators und die Länge des ersten Festbetts entlang der Strömungsrichtung des Einsatzstoffstromes bzw. des Produktstoffstromes sowie der Gehalt des verwendeten Feed-Alkans (z.B. Ethan) im Einsatzstoffstrom zum ersten Festbett so bemessen, dass der adiabatische Temperaturanstieg höchste Umsätze bei höchster Selektivität ermöglicht, wobei in einem nachgeschalteten zweiten Festbett auf eine Verdünnung des Katalysators verzichtet werden kann, um den vollständigen Umsatz des verwendeten Alkans (z.B. Ethan) zu ermöglichen (siehe unten).

Bei isothermer oxidativer Dehydrierung wird das mindestens eine Festbett bevorzugt mittels einer Flüssigkeit, eines Wärmeträgeröls oder einer Salzschmelze gekühlt. Im Falle einer adiabatischen oxidativen Dehydrierung wird der Einsatzstoffstrom bevorzugt durch eine Mehrzahl an aufeinanderfolgenden Festbetten gegeben, wobei bevorzugt der Einsatzstoffstrom zwischen je zwei benachbarten Festbetten zwischengekühlt wird, insbesondere durch Einspritzen von Wasser, und/oder dem Einsatzstoffstrom Sauerstoff, Stickstoff und/oder Luft hinzugegeben wird, wobei insbesondere in der Luft Sauerstoff oder Stickstoff an- oder abgereichert sein kann.

Bei der besagten Flüssigkeit zum Kühlen des mindestens einen Festbetts kann es sich insbesondere um Wasser oder einen Kohlenwasserstoff oder ein Gemisch der vorgenannten Stoffe handeln. Bei einem Kohlenwasserstoff kann es sich dabei um einen nicht-funktionalisierten Kohlenwasserstoff oder um einen funktionalisierten Kohlenwasserstoff (z.B. Alkohol) handeln. Die besagten Flüssigkeiten können beim Kühlen sieden.

Weitere Einzelheiten und Vorteile der Erfindung sollen durch die nachfolgenden Figurenbeschreibungen von Ausführungsbeispielen anhand der Figuren erläutert werden.

Es zeigen:
- Fig. 1: eine schematische Schnittdarstellung einer erfindungsgemäßen, isotherm betriebenen Reaktoreinrichtung;
- Fig. 2: eine schematische Schnittdarstellung einer weiteren, adiabatisch betriebenen Reaktoreinrichtung;
- Fig. 3: ein Blockdiagramm eines erfindungsgemäßen Verfahrens zur oxidativen Dehydrierung von Alkanen (z.B. Ethan), bei dem eine Verdünnung des Einsatzstoffstromes mit Wasserdampf erfolgt;
- Fig. 4: ein Blockdiagramm eines weiteren erfindungsgemäßen Verfahrens zur oxidativen Dehydrierung von Alkanen (z.B. Ethan), bei dem eine Verdünnung des Einsatzstoffstromes mit Stickstoff erfolgt; und
- Fig. 5: ein Blockdiagramm eines weiteren erfindungsgemäßen Verfahrens zur oxidativen Dehydrierung von Alkanen (z.B. Ethan), bei dem eine Verdünnung des Einsatzstoffstromes mit Wasserdampf und Stickstoff erfolgt.

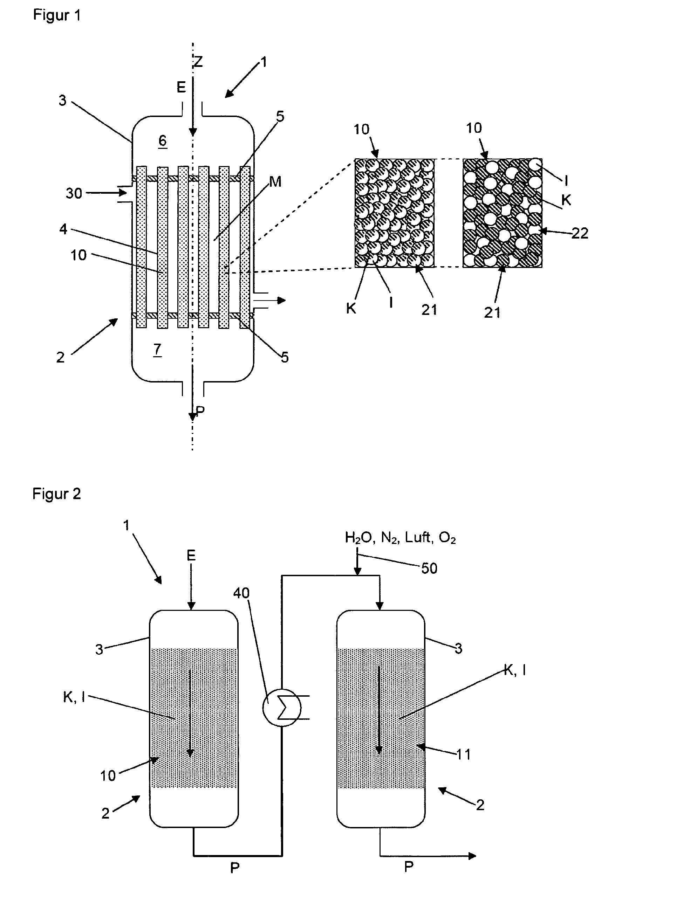

Figur 1 zeigt eine erfindungsgemäße Reaktoreinrichtung 1 mit zumindest einem Reaktor 2 zur isothermen Reaktionsführung. Der Reaktor 2 weist einen Reaktormantel 3 auf, der einen Mantelraum M zur Aufnahme eines Kühlmediums 30 in Form einer Flüssigkeit oder eines sonstigen geeigneten Kühlmediums 30 umschließt, sowie eine Mehrzahl an im Mantelraum M angeordneten Reaktorrohren 4, die jeweils ein Festbett 10 aufnehmen, das einen Katalysator K für die oxidative Dehydrierung von Ethan zu Ethylen aufweist. Die Reaktorrohre 4 sind von dem Kühlmedium 30 umgeben, so dass die Reaktionswärme der oxidativen Dehydrierung zur isothermen Reaktionsführung vom Kühlmedium 30 abgeführt werden kann. Die Reaktorrohre 4 sind dabei in Rohrböden 5 verankert, die vom Mantelraum M eine Einlass- sowie eine Auslasskammer 6, 7 abtrennen, wobei über die Einlasskammer 6 ein ethan- und sauerstoffhaltiger Einsatzstoffstrom E durch die Festbetten 10 entlang der Vertikale Z von oben nach unten durchgeleitet wird. In den einzelnen Festbetten 10 entsteht dabei durch oxidatives Dehydrieren des Ethans ein ethylenhaltiger Produktstoffstrom P, der über die Auslasskammer 7 aus dem Reaktor 2 abgezogen wird.

Wie in Figur 1 (Detail mitte) gezeigt, besteht in einer ersten Variante das jeweilige Festbett 10 aus zumindest einer Mehrzahl an den Katalysator K aufweisenden ersten Partikeln 21, die zum Verdünnen des Katalysators K des Weiteren ein Inertmaterial I aufweisen, bei dem es sich z.B. um Aluminiumoxid, Siliciumdioxid, Siliciumcarbid, Quartz oder Keramik handeln kann.

Gemäß einer weiteren Variante (Detail Figur 1 rechts) ist vorgesehen, dass das jeweilige Festbett 10 zum Verdünnen des Katalysators K eine Mehrzahl an mit den ersten Partikeln 21 regellos vermischten zweiten Partikeln 22 aufweist, die aus dem Inertmaterial I gebildet sind. Hierbei sind die Partikel 21, 22 so vermischt, dass in dem jeweiligen Festbett 10 pro Volumeneinheit eine konstante Dichte an ersten bzw. zweiten Partikeln 21, 22 herrscht.

Durch die Verdünnung des Katalysators K mit Inertmaterial I entsteht pro Volumeneinheit in den Reaktorrohren 4 weniger Wärme, die durch das Kühlmedium 30 auf der Außenseite leichter abgeführt werden kann. Der Grad der Verdünnung wird so bemessen, dass die Reaktionstemperatur in den Reaktorrohren 4 so hoch gehalten werden kann, dass größtmögliche Umsätze und Selektivitäten erreicht werden, ohne dass es zu einem Durchgehen der Reaktion führt. Das Inertmaterial I bzw. die zweiten Partikel 22 können dabei auch eine andere Form oder eine andere Größe aufweisen als der aktive Katalysator K bzw. die ersten Partikel 21, um dadurch den Druckabfall beim Durchströmen des Festbetts 10 bzw. der Schüttung 10 klein zu halten.

### Beispiel 1

Prozessdaten für die Reaktoreinrichtung 1 in Form eines Isotherm reaktors gemäß Figur 1:
- Druck im Reaktor 2: von 0,5 bar bis 35 bar, bevorzugt 1 bar bis 15 bar, besonders bevorzugt 1 bar bis 5 bar.
- Temperatur im Reaktor 2: zwischen 250°C bis 650°C, bevorzugt 280°C bis 550°C, besonders bevorzugt 350°C bis 480°C.
- Feedzusammensetzungen (Einsatzstoffstrom E): 1 Vol.-% bis 10 Vol.-% Ethan, 1 Vol.-% bis 5 Vol.-% O₂, 10 Vol.-% bis 95 Vol.-% H₂O, Rest N₂, bevorzugt 1 Vol.-% bis 5 Vol.-%, 1 Vol.-% bis 3 Vol.-% O₂, 20 Vol.-% bis 90 Vol.-% H₂O, Rest N₂, besonders bevorzugt 1 Vol.-% bis 3 Vol.-% Ethan, 1 Vol.-% bis 3 Vol.-% O₂, 25 Vol.-% bis 90 Vol.-% H₂O, Rest N₂.
- WHSV = 1.0 kg bis 20 kg C₂H₆/h/kgKat, bevorzugt WHSV = 5kg bis 15 kg C₂H₆/h/kgKat, besonders bevorzugt WHSV = 5 kg bis 10 kg C₂H₆/h/kgKat.
- Der Anteil des Inertmaterials I an den Festbetten 10 beträgt 30 Vol.-% bis 90 Vol.-%, bevorzugt 50 Vol.-% bis 80 Vol.-%, besonders bevorzugt 60 Vol.-% bis 80 Vol.-%.

Gemäß Figur 2 kann die erfindungsgemäße Reaktoreinrichtung 1 auch adiabatisch betrieben werden. Hierbei sind bevorzugt mehrere Festbetten 10, 11 der oben beschriebenen Art (insbesondere Verdünnung mit Inertmaterial I) hintereinander geschaltet, so dass der Einsatzstoffstrom E bzw. Produktstoffstrom P diese nacheinander durchlaufen kann. Hierbei wird zwischen zwei Festbetten 10, 11 eine Zwischenkühlung des aus dem jeweiligen vorangegangenen Festbett 10 ausgetretenen Stoffstromes P vorgenommen, wobei des Weiteren über einen Einlass 50 stromauf der nächsten Stufe bzw. stromauf des nächsten Festbettes 11 eine Zwischeneinspeisung von Luft oder Sauerstoff vorgenommen wird. In der Figur 2 sind exemplarisch lediglich zwei Festbetten 10, 11 gezeigt. Es können natürlich auch mehr als zwei Festbetten 10, 11 vorhanden sein. Die Festbetten 10, 11 können des Weiteren in separaten Reaktoren 2 bzw. Reaktormänteln 3 angeordnet sein.

Der Grad der Verdünnung des aktiven Katalysators K und die Länge des ersten Festbettes 10 sowie der Gehalt an Ethan im Einsatzstoffstrom bzw. Gaszustrom E zum ersten Festbett 10 wird dabei so bemessen, dass der adiabatische Temperaturanstieg höchste Umsätze bei höchster Selektivität ermöglicht. In dem nachgeschalteten zweiten Festbett 11 oder mindestens einem weiteren Festbett kann auf eine Verdünnung des aktiven Katalysators K verzichtet werden, um den vollständigen Umsatz von Ethan zu ermöglichen.

### Beispiel 2

Prozessdaten für adiabatische Reaktoreinrichtung 1 gemäß Figur 2 mit Zwischenkühlern 40 vor dem nächsten Festbett 11 :
- Druck in Reaktoren 2: von 0,5 bar bis 35 bar, bevorzugt 1 bar bis 15 bar, besonders bevorzugt 1 bar bis 5 bar.
- Temperatur in Reaktoren 2: zwischen 250°C bis 650°C, bevorzugt 280°C bis 550°C, besonders bevorzugt 350°C bis 480°C.
- Feedzusammensetzungen (Einsatzstoffstrom E): 1 Vol.-% bis 10 Vol.-% Ethan, 1 Vol.-% bis 5 Vol.-% O₂, 10 Vol.-% bis 95 Vol.-% H₂O, Rest N₂, bevorzugt 1 Vol.-% bis 5 Vol.-% Ethan, 1 Vol.-% bis 3 Vol.-% O₂, 20 Vol.-% bis 90 Vol.-% H₂O, Rest N₂, besonders bevorzugt 2 Vol.-% bis 4 Vol.-% Ethan, 1 Vol.-% bis 3 Vol.-% O₂, 25 Vol.-% bis 90 Vol.-% H₂O, Rest N₂
- WHSV = 1 kg bis 40 kg C₂H₆/h/kgKat, bevorzugt WHSV = 2 kg bis 30 kg C₂H₆/h/kgKat, besonders bevorzugt WHSV = 5 kg bis 25 kg C₂H₆/h/kgKat.
- Der Anteil des Intermaterials I an den Festbetten 10, 11 beträgt 30 Vol.-% bis 90 Vol.-%, bevorzugt 50 Vol.-% bis 85 Vol.%, besonders bevorzugt 60 Vol.-% bis 80 Vol.-%. Das zweite Festbett 11 oder mindestens ein weiteres Festbett kann ohne Intertmaterial I ausgeführt sein.

Für beide Reaktionsführungen (isotherm und adiabatisch) kann das Inertmaterial I auch in den Katalysator K eingearbeitet sein (siehe oben). Die entsprechenden Partikel bzw. Formkörper 21 (Pellets, Extrudat oder andere) können also aus einer Mischung aus Inert- und Aktivmaterial K erzeugt werden.

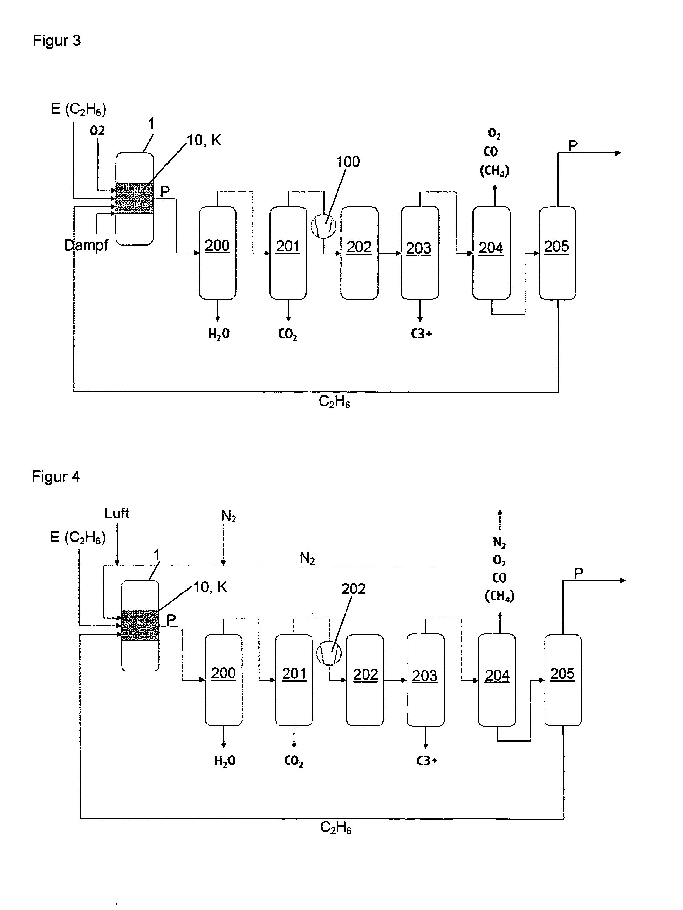

Figur 3 zeigt eine weitere Variante des erfindungsgemäßen Verfahrens zur oxidativen Dehydrierung von Alkanen, hier in Form von Ethan.

Danach werden als Einsatzgase (Einsatzstoffstrom E) ein Alkan, vorliegend Ethan, sowie Sauerstoff als Oxidationsmittel in einer Reaktoreinrichtung 1 einem Katalysator K zugeführt, bei dem es sich bevorzugt um einen MoVTeNbOₓ-Katalysator handelt, wobei das Ethan oxidativ unter Entstehung eines ethylenhaltigen Produktstoffstromes P in Gegenwart des Katalysators K dehydriert wird (anstelle von Ethan kommen als Einsatz insbesondere auch Propan und/oder Butan in Frage), wobei zur kontrollierten Reaktion außerhalb von Explosionsbereichen ein inertes Verdünnungsmittel in die Reaktoreinrichtung 1 in Form von Wasserdampf eingeleitet wird. Als Reaktoreinrichtung 1 kann prinzipiell eine Reaktoreinrichtung 1 gemäß Figur 1 oder 2 verwendet werden. Weiterhin kann der nachfolgend beschriebene Zerlegungsprozess auch dem hinsichtlich der Figuren 1 und 2 beschriebenen Verfahren nachgeschaltet werden.

Der ethylenhaltige Stoffstrom P wird nun aus der Reaktoreinrichtung 1 abgezogen und in einer entsprechenden Kolonne einer Wasserwäsche 200 zugeführt , die als erste Reinigungsstufe (insbesondere Auswaschen wasserlöslicher Bestandteile wie z.B. Carbonsäure, insbesondere Essigsäure, und/oder Carbonylverbindungen, wie z.B. Aldehyde oder Ketone) des Produktstoffstromes P dient, wobei das auskondensierte Wasser aus dem Sumpf der Kolonne 200 abgezogen und als Makeupwasser der Wäsche genutzt wird.

Der solchermaßen vorgereinigte Produktstoffstrom P wird sodann insbesondere zum Entfernen von bei der oxidativen Dehydrierung entstandenem CO₂ einer Amin- und/oder Laugewäsche 201 unterzogen, wonach der Produktstoffstrom P in einem Verdichter 100 verdichtet und in einer Adsorberstation 202 getrocknet wird.

Hiernach wird der Produktstoffstrom P in eine Deethanisierer-Kolonne 203 (auch als Deethanizer bezeichnet) eingespeist, wobei ein C₂₋-Strom enthaltend u.a. Ethan und Ethylen über Kopf abgezogen wird und einer Demethanisierer-Kolonne (auch als Demethanizer bezeichnet) 204 zugeführt wird. Der C₃₊-Strom wird aus dem Sumpf des Deethanizers 203 abgezogen.

Aus dem Demethanizer 204 werden dann über Kopf die Komponenten O₂, CO und CH₄ abgezogen, wobei der entstehende ethan- und ethylenhaltige Produktstoffstrom P in einen C2-Splitter 205 gegeben wird, aus dem über Kopf das Ethylenprodukt P abgezogen wird. Das aus dem Sumpf des C2-Splitters abgezogene Ethan wird in die Reaktoreinrichtung 1 als Einsatz zurückgeführt.

In einer in Figur 4 gezeigten Abwandlung des Verfahrens gemäß Figur 3 wird anstelle von Sauerstoff Luft als Verdünnungs- bzw. Oxidationsmittel in die Reaktoreinrichtung 1 eingeleitet. Dabei kann Stickstoff und/oder Sauerstoff in der Luft an- oder abgereichert sein. Durch Reaktion des in der Luft enthaltenen Sauerstoffes verbleiben Stickstoff und inerte Komponenten aus der Luft im Produktstrom P. Der Stickstoff und Inerte werden so dann im Zerlegungsteil 204 abgetrennt und als Verdünnungsmittel in die Reaktoreinrichtung 1 zurückgeführt. Lediglich bei Inbetriebnahme (Startup) ist ggf. die Zuführung eines Stickstoff-Makeupstromes erforderlich. Angesammelte Verunreinigungen können über einen Purge-Strom ausgeschleust werden. Je nach Erfordernis kann eine zusätzliche Einspeisung von reinem N₂ erfolgen, um die Sauerstoffkonzentration gering zu halten und / oder hohe Purgeraten zu ermöglichen.

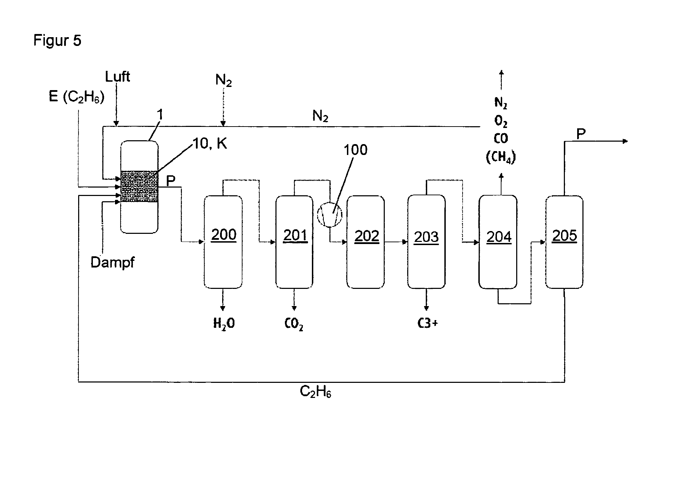

Weiterhin zeigt Figur 5 eine Kombination der Verfahren gemäß Figuren 3 und 4. Danach wird zusätzlich zum Stickstoff Wasserdampf als Verdünnungsmittel in die Reaktoreinrichtung 1 eingeleitet. Auf diese Weise können die Vorteile der Varianten gemäß Figuren 3 und 4 kombiniert werden.

Grundsätzlich kann beim Anfahren der Anlage Wasserdampf oder Stickstoff oder ein Gemisch dieser beiden Stoffe als Verdünnungsmittel in die Reaktoreinrichtung 1 eingeleitet werden. Sodann werden die Reaktanden für die oxidative Dehydrierung in die Reaktoreinrichtung 1 gegeben, also das verwendete Alkan sowie das verwendete Oxidationsmittel, wobei das Oxidationsmittel in Form von Sauerstoff oder Luft hinzugegeben werden kann. In der Luft kann dabei Stickstoff und/oder Sauerstoff jeweils an- oder abgereichert sein. In die Reaktoreinrichtung 1 gegebener Stickstoff wird, wie oben beschrieben, nach Abtrennung bevorzugt in die Reaktoreinrichtung 1 zurückgeführt.

Nach dem Anfahren kann das beim Anfahren hinzugegebene Verdünnungsmittel weiterhin in die Reaktoreinrichtung 1 eingeleitet werden oder zumindest teilweise oder auch vollständig durch ein anderes der genannten Verdünnungsmittel ersetzt werden. Ein anderes der genannten Verdünnungsmittel kann auch zusätzlich in die Reaktoreinrichtung 1 eingeleitet werden.

### Beispiel 3

Prozessdaten für das Verfahren zur oxidativen Dehydrierung gemäß Figuren 3 bis 5:
- Druck in Reaktoreinrichtung 1: von 0,5 bar bis 35 bar, bevorzugt 1 bar bis 15 bar, besonders bevorzugt 1 bar bis 5 bar.
- Temperatur in Reaktoreinrichtung 1: 250°C bis 650°C, bevorzugt 280°C bis 550°C, besonders bevorzugt 350°C bis 480°C.
- Feed (Einsatzstoffstrom E): n-Alkane, bevorzugt C2-C6-Alkane, besonders bevorzugt Ethan.
- Zuführung eines Verdünnungsmittels bzw. Intertmediums (Wasserdampf, Stickstoff, Edelgase, andere unter Reaktionsbedingungen inerte Gase, sowie beliebige Mischungen davon), bevorzugt sind Wasser und Stickstoff bzw. deren Mischungen.
- Verwendung von reinem Sauerstoff oder Luft als Oxidationsmittel.

Die Verfahrensvarianten gemäß Figuren 3 bis 5 können mit einem beliebigen Katalysator durchgeführt werden, der unter den Reaktionsbedingungen stabil ist. Bevorzugt wird vorliegend jedoch ein MoVTeNbOₓ-Katalysator verwendet.

### Bezugszeichenliste

| | |
|---|---|
| 1 | Reaktoreinrichtung |
| 2 | Reaktor |
| 3 | Reaktormantel bzw. Reaktorrohr |
| 4 | Reaktorrohr |
| 5 | Rohrboden |
| 6 | Einlasskammer |
| 7 | Auslasskammer |
| 10, 11 | Festbett |
| 21 | Erste Partikel (Schüttfüllkörper) |
| 22 | Zweite Partikel (Schüttfüllkörper) |
| 30 | Kühlmedium (z.B.siedendes Wasser) |
| 40 | Einrichtung zum Kühlen |
| 50 | Einlass (Zwischeneinspeisung) |
| 100 | Verdichter |
| 200 | Wasserwäsche(-Kolonne) |
| 201 | Waschkolonne (Amin- und/oder Laugenwäsche) |
| 202 | Trocknung |
| 203 | Deethanisierer |
| 204 | Demethanisierer |
| 205 | C2-Splitter |
| E | Einsatzstoffstrom |
| I | Inertmaterial |
| K | (aktiver) Katalysator |
| M | Mantelraum |
| P | Produktstoffstrom |
| Z | Vertikale |

## Patentansprüche

1. Verfahren zur oxidativen Dehydrierung von Alkanen, insbesondere von Ethan zu Ethylen, bei dem ein Einsatzstoffstrom (E) enthaltend ein Alkan, insbesondere Ethan, in einer Reaktoreinrichtung (1) einem Katalysator (K) zugeführt wird, wobei durch oxidatives Dehydrieren des Alkans mit Sauerstoff in Gegenwart des Katalysators (K) ein alkenhaltiger Produktstoffstrom (P) erzeugt wird, und wobei ein Verdünnungsmittel in die Reaktoreinrichtung (1) eingeleitet wird, das inert ist oder zumindest eine inerte Komponente aufweist.

2. Verfahren nach Anspruch 1, **dadurch gekennzeichnet, dass** das Verdünnungsmittel in die Reaktoreinrichtung (1) eingeleitet wird, um die Wärmetönung bei der oxidativen Dehydrierung des Alkans zu kontrollieren, insbesondere um eine Explosion bei der oxidativen Dehydrierung des Alkans zu verhindern.

3. Verfahren nach Anspruch 1 oder 2, **dadurch gekennzeichnet, dass** als Verdünnungsmittel einer der folgenden Stoffe oder eine Kombination mehrerer der folgenden Stoffe verwendet wird:
- Wasserdampf,
- Stickstoff,
- Luft.

4. Verfahren nach einem der vorhergehenden Ansprüche, **dadurch gekennzeichnet, dass** Luft zur Bereitstellung von Sauerstoff als Oxidationsmittel in die Reaktoreinrichtung (1) eingeleitet wird.

5. Verfahren nach Anspruch 3 oder 4, **dadurch gekennzeichnet, dass** Stickstoff in besagter Luft angereichert oder abgereichtert ist, und/oder dass Sauerstoff in besagter Luft angereichert oder abgereichert ist.

6. Verfahren nach einem der vorhergehenden Ansprüche, **dadurch gekennzeichnet, dass** im Produktstoffstrom (P) enthaltener Stickstoff und/oder enthaltene sonstige Inerte abgetrennt werden und als Verdünnungsmittel in die Reaktoreinrichtung (1) zurückgeführt werden.

7. Verfahren nach einem der vorhergehenden Ansprüche, **dadurch gekennzeichnet, dass** beim Anfahren Wasserdampf und/oder Stickstoff als Verdünnungsmittel in die Reaktoreinrichtung (1) gegeben wird und sodann besagtes Alkan und als Oxidationsmittel der Sauerstoff oder den Sauerstoff enthaltene Luft hinzugegeben werden, wobei insbesondere Sauerstoff und/oder Stickstoff in besagter Luft jeweils an- oder abgereichert ist.

8. Verfahren nach Anspruch 7, **dadurch gekennzeichnet, dass** das beim Anfahren hinzugegebene Verdünnungsmittel nach dem Anfahren weiterhin in die Reaktoreinrichtung (1) gegeben wird oder zumindest teilweise oder vollständig durch ein anderes Verdünnungsmittel ersetzt wird.

9. Verfahren nach einem der vorhergehenden Ansprüche, **dadurch gekennzeichnet, dass** die oxidative Dehydrierung in der Reaktoreinrichtung (1) bei einem Druck von 0,5 bar bis 35 bar, bevorzugt 1 bar bis 15 bar, besonders bevorzugt 1 bar bis 5 bar durchgeführt wird.

10. Verfahren nach einem der vorhergehenden Ansprüche, **dadurch gekennzeichnet, dass** die oxidative Dehydrierung in der Reaktoreinrichtung (1) bei einer Temperatur von 250°C bis 650°C, bevorzugt 280°C bis 550°C, besonders bevorzugt 350°C bis 480°C durchgeführt wird.

11. Verfahren nach einem der vorhergehenden Ansprüche, **dadurch gekennzeichnet, dass** es sich bei dem Alkan um ein C2 bis C6-Alkan handelt, bevorzugt um Ethan.

12. Verfahren nach einem der vorhergehenden Ansprüche, **dadurch gekennzeichnet, dass** als Katalysator (K) ein MoVTeNbOₓ-Katalysator verwendet wird.

13. Verfahren nach einem der vorhergehenden Ansprüche, **dadurch gekennzeichnet, dass** der Katalysator (K) mit einem Inertmaterial (I) verdünnt ist.

14. Verfahren nach Anspruch 13, **dadurch gekennzeichnet, dass** die Reaktoreinrichtung (1) zumindest ein den Katalysator (K) aufweisendes Festbett (10, 11) aufweist, wobei insbesondere das mindestens eine Festbett (10, 11) aus zumindest einer Mehrzahl an jenen Katalysator (K) aufweisenden ersten Partikeln (21) gebildet ist, wobei insbesondere jene ersten Partikel (21) auch das Inertmaterial (I) aufweisen und/oder wobei insbesondere das mindestens eine Festbett (10, 11) zum Verdünnen des Katalysators (K) eine Mehrzahl an mit den ersten Partikeln (21) vermischte zweite Partikel (22) aufweist, die aus dem Inertmaterial (I) gebildet sind.

15. Verfahren nach Anspruch 14, **dadurch gekennzeichnet, dass** die oxidative Dehydrierung isotherm erfolgt, wobei das mindestens eine Festbett (10) mittels einer Flüssigkeit (30), eines Wärmeträgeröls oder einer Salzschmelze gekühlt wird, und/oder dass die oxidative Dehydrierung adiabatisch erfolgt, wobei insbesondere der Einsatzstoffstrom (E) durch eine Mehrzahl an aufeinanderfolgenden Festbetten (10, 11) gegeben wird, wobei insbesondere der Einsatzstoffstrom (E) zwischen je zwei benachbarten Festbetten (10, 11) zwischengekühlt wird, insbesondere durch Einspritzen von Wasser, und/oder dem Einsatzstoffstrom (E) Sauerstoff, Stickstoff und/oder Luft hinzugegeben wird, wobei insbesondere in der Luft Sauerstoff und/oder Stickstoff an- oder abgereichert ist.
